# DEMANDE DE BREVET EUROPEEN

(11) **EP 1 459 730 A1**
(43) Date de publication de la demande: **22.09.2004**
(21) Numéro de dépôt: 04290624.8
(22) Date de dépôt: 08.03.2004
(51) Int. Cl.: A61K 7/043

(54) **Composition de vernis à ongles comprenant des fibres recouvertes d'au moins un azurant optique**

(30) Priorité: 19.03.2003 FR 0303355
(71) Demandeur: L'OREAL, 75008 Paris (FR)
(72) Inventeur: Ramin, Roland, 75014 Paris (FR)
(74) Mandataire: Boulard, Denis

(57) **Abrégé**

L'invention a pour objet une composition de vernis à ongles comprenant, dans un milieu cosmétiquement acceptable, des fibres recouvertes d'au moins un azurant optique.

## Description

La présente invention a pour objet un vernis à ongles comprenant des fibres recouvertes d'au moins un azurant optique. L'invention a également pour objet un procédé de maquillage ou de soin des ongles.

La composition peut être employée comme produit de maquillage des ongles tel qu'un vernis à ongles coloré, comme base pour les ongles ou "base-coat" en terminologie anglo-saxonne, comme composition de finition, encore appelée "top-coat", à appliquer sous ou sur le produit de maquillage des ongles ou bien encore comme produit de soin cosmétique des ongles tel qu'une base traitante destinée à protéger, à renforcer et/ou réparer les ongles.
Cette composition peut s'appliquer sur les ongles d'êtres humains ou bien encore sur des faux ongles. De préférence, la composition selon l'invention est une base traitante pour vernis à ongles.

Il est connu des formulateurs d'utiliser des fibres dans des vernis à ongles, par exemple pour réparer et renforcer les ongles cassés comme proposé dans le document FR-A-1529329. On peut également citer le document EP-B-583347 qui décrit des vernis à ongles comprenant des fibres d'aramide qui permettent l'obtention d'une bonne adhérence du vernis sur l'ongle, ou encore le document WO-A-02/41852 qui propose un vernis à ongles ayant un toucher velouté et un aspect visuel brillant grâce à l'utilisation de fibres plates.

Cependant, l'utilisation de fibres dans des compositions de vernis à ongles se caractérise souvent par une mauvaise dispersion des fibres, qui se traduit par un dépôt sur l'ongle non homogène et d'aspect non lisse et granuleux.
C'est pourquoi on cherche à obtenir une composition de vernis à ongles comprenant des fibres susceptible de renforcer les ongles tout en permettant l'obtention d'un film d'aspect visuel satisfaisant pour l'utilisatrice.

Le demandeur a constaté de façon surprenante qu'une composition de vernis à ongles comprenant des fibres recouvertes d'au moins un azurant optique permet d'obtenir les propriétés décrites ci-dessus.

Une telle composition permet l'obtention, après application sur les ongles, d'un dépôt d'aspect homogène, régulier, qui camoufle les imperfections des ongles (notamment striés, fendus, dédoublés) grâce aux fibres revêtues d'azurant optique et leur confère un aspect sain. Le film déposé sur l'ongle présente de bonnes propriétés de tenue et de résistance mécanique, il est résistant aux frottements, aux chocs, aux rayures.

De façon plus précise, l'invention a pour objet une composition de vernis à ongles comprenant, dans un milieu physiologiquement acceptable, des fibres recouvertes d'au moins un azurant optique.

L'utilisation d'azurant optique dans des compositions de vernis à ongles est connue, par exemple du document FR-A-2 741 261 qui décrit des compositions cosmétiques comprenant un agent fluorescent d'avivage, également connu comme azurant optique, pour accroître l'intensité apparente de la couleur d'un vernis à ongles. Le document FR-A-2773470 propose également l'utilisation d'un additif fluorescent dans des produits de maquillage des ongles qui permet de neutraliser l'effet jaune donné par les résines ou qui présente un reflet bleu fluorescent sous un éclairage.

Aucun de ces documents ne divulgue ni ne suggère une composition contenant des fibres recouvertes d'azurant optique telle que la composition de la présente invention.

L'invention a aussi pour objet un procédé cosmétique de maquillage ou de soin non thérapeutique des ongles comprenant l'application sur les ongles d'au moins une couche d'une composition de vernis à ongles telle que décrite ci-dessus.

L'invention a encore pour objet l'utilisation de fibres recouvertes d'au moins un azurant optique dans une composition de vernis à ongles, pour conférer au dépôt obtenu après l'application de la dite composition sur les ongles une bonne tenue et/ou une résistance mécanique du film de composition et/ou un effet renforçateur de l'ongle.

L'invention a encore pour objet l'utilisation de fibres recouvertes d'au moins un azurant optique dans une composition de vernis à ongles, pour conférer au dépôt obtenu après l'application de la dite composition sur les ongles un aspect homogène et/ou régulier et/ou un camouflage des imperfections des ongles.

Par "milieu cosmétiquement acceptable", on entend au sens de l'invention un milieu compatible avec le maquillage des ongles.

Par "fibre", il faut comprendre un objet de longueur L et de diamètre D tel que L soit très supérieur à D, D étant le diamètre du cercle dans lequel s'inscrit la section de la fibre. En particulier, le rapport UD (ou facteur de forme) est choisi dans la gamme allant de 3,5 à 2500, de préférence de 5 à 500, et mieux de 5 à 150.

Les fibres utilisées dans la composition selon l'invention sont des fibres disponibles dans le commerce qui sont déjà recouvertes d'azurant optique ou bien des fibres non recouvertes d'azurant optique et qui sont ensuite recouvertes d'azurant optique par des procédé connus de l'homme du métier.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

Les fibres peuvent avoir une longueur allant de 1 nm à 10 mm. Parmi ces fibres, on peut en particulier distinguer les fibres dites" nanométriques" qui présentent une longueur allant de 1 nm à 999 nm. En particulier, les fibres ont une longueur allant de 1 µm à 10 mm, de préférence de 0,1 mm à 5 mm et mieux de 0,3 mm à 1 mm. Leur section peut être comprise dans un cercle de diamètre allant de 2 nm à 500 µm, de préférence allant de 100 nm à 100 µm et mieux de 1 µm à 50 µm. Le poids ou titre des fibres est souvent donné en denier ou décitex et représente le poids en gramme pour 9 km de fil. De préférence, les fibres selon l'invention ont un titre choisi dans la gamme allant de 0,01 à 10 à deniers, de préférence de 0,1 à 2 deniers et mieux de 0,3 à 0,7 deniers.

Les fibres peuvent être celles utilisées dans la fabrication des textiles et notamment des fibres de soie, de coton, de laine, de lin, des fibres de cellulose - notamment extraites notamment du bois, des légumes ou des algues -, de rayonne, de polyamide (Nylon®), de viscose, d'acétate notamment d'acétate de rayonne, de poly-(p-phénylène-téréphtalamide) (ou d'aramide) notamment de Kevlar®, de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.
De préférence, les fibres sont des fibres de polyamide ((Nyton®).

On peut aussi utiliser les fibres utilisées en chirurgie comme les fibres synthétiques résorbables préparées à partir d'acide glycolique et caprolactone ("Monocryl" de chez Johnson & Johnson) ; les fibres synthétiques résorbables du type copolymère d'acide lactique et d'acide glycolique ("Vicryl" de chez Johnson & Johnson) ; les fibres de polyester téréphtalique ("Ethibond" de chez Johnson & Johnson) et les fils d'acier inoxydable ("Acier" de chez Johnson & Johnson).

Par ailleurs, outre leur revêtement d'azurant optique, les fibres peuvent être traités ou non en surface, enrobées ou non d'une couche de protection. Cet enrobage ou traitement de surface se situe sous le revêtement d'azurant optique si le matériau constitutif de la couche de protection est opaque ou semi-opaque ; il peut se situer au dessus du revêtement d'azurant optique si ce matériau est transparent ou semi-transparent.

Comme fibres enrobées utilisables dans l'invention, on peut citer des fibres de polyamides enrobées de sulfure de cuivre pour un effet anti-statique (par exemple le "R-STAT" de chez Rhodia) ou un autre polymère permettant une organisation particulière des fibres (traitement de surface spécifique). On peut encore citer les fibres enrobées par des pigments minéraux
ou organiques, tels que les pigments cités plus loin dans la demande.

De préférence, on utilise des fibres d'origine synthétique et en particulier des fibres organiques, comme celles utilisées en chirurgie.

Les fibres utilisables dans la composition selon l'invention sont préférentiellement des fibres de polyamide, de cellulose, de poly-p-phénylène téréphtalamide ou de de polyéthylène. Leur longueur (L) peut aller de 0,1 mm à 5 mm, de préférence de 0,25 mm à 1,6 mm et leur diamètre moyen peut aller de 1 µm à 50 µm. En particulier, on peut utiliser les fibres de polyamide commercialisées par les Etablissements P. Bonte sous le nom de "Polyamide 0.9 Dtex 3 mm", ayant un diamètre moyen de 6 µm, un titre d'environ 0.9 dtex et une longueur allant de 0,3 mm à 5 mm. On peut aussi utiliser les fibres de celluloses (ou de rayonne) ayant un diamètre moyen de 50 µm et une longueur allant de 0,5 mm à 6 mm comme celles vendues sous le nom de "Natural rayon flock fiber RC1BE - N003 - M04" par la société Claremont Flock. On peut également utiliser des fibres de polyéthylène comme celles vendues sous le nom de "Shurt Stuff 13 099 F" par la société Mini Fibers.
De préférence, on utilise les fibres recouvertes d'azurant optique telles que commercialisées par la société LCW sous la référence commerciale Fiberlon 54 ZO3, ayant une longueur d'environ 0,4 mm et un titre de 0,5 deniers.

La composition selon l'invention peut également comprendre des fibres dites "rigides", sensiblement rectilignes, par opposition aux fibres citées précédemment, qui ne sont pas des fibres rigides.
Les fibres rigides, initialement sensiblement droites, lorsqu'elles sont placées dans un milieu dispersant, ne voient pas leur forme sensiblement modifiée, ce qui se traduit par la condition angulaire définie ci-après, reflétant une forme que l'on peut qualifier de toujours, sensiblement droite, linéaire. Cette condition d'angle reflète la rigidité des fibres qui peut difficilement être exprimée par un autre paramètre pour des objets ayant une taille aussi faible que les fibres rigides.

La rigidité des fibres se traduit par la condition angulaire suivante : avantageusement, au moins 50 % en nombre, de préférence au moins 75 % en nombre, et mieux au moins 90 % en nombre des fibres sont telles que l'angle formé entre la tangente à l'axe central longitudinal de la fibre et la droite reliant l'une des extrémités au point sur l'axe central longitudinal de la fibre correspondant à la moitié de la longueur de la fibre soit inférieur à 15° et l'angle formé entre la tangente à l'axe central longitudinal de la fibre en un point situé à mi-longueur de la fibre et la droite reliant l'une des extrémités au point sur l'axe central longitudinal de la fibre correspondant à la moitié de la longueur de fibre soit inférieur ou égal à 15 °, pour une même longueur de fibre allant de 0,8 mm à 5 mm, de préférence allant de 1 mm à 4 mm, de préférence allant de 1 mm à 3 mm, et mieux de 2 mm.
Avantageusement, l'angle, mentionné ci-dessus, est mesuré aux deux extrémités de la fibre et en un point situé à mi-longueur de la fibre, en d'autres termes, on effectue dans ce cas trois mesures et la moyenne des angles mesurés est inférieure ou égale à 15°.
Notamment, la tangente, en tout point de la fibre, forme un angle inférieur à 15°.
Dans la présente demande, l'angle formé par la tangente en un point de la fibre est l'angle formé entre la tangente à l'axe central longitudinal de la fibre audit point de la fibre et la droite reliant l'extrémité de la fibre la plus proche dudit point au point sur l'axe central longitudinal de la fibre correspondant à la moitié de la longueur de la fibre.

Généralement, les fibres rigides utilisables dans la composition selon l'invention ont la même longueur de fibre ou une longueur sensiblement identique.

Plus précisément lorsque l'on observe au microscope, avec un objectif permettant un grossissement de 2,5 et avec une vision plein champ, un milieu dans lequel sont dispersées les fibres rigides à une concentration des fibres de 1 % en poids, un nombre majoritaire de fibres rigides, c'est-à-dire au moins 50 % en nombre des fibres rigides, de préférence au moins 75 % en nombre des fibres rigides, et mieux au moins 90 % en nombre des fibres rigides, doivent satisfaire à la condition angulaire définie plus haut. La mesure conduisant à la valeur de l'angle est effectuée pour une même longueur de fibres, cette longueur est comprise dans la gamme allant de 0,8 mm à 5 mm, de préférence de 1 à 4 mm, de préférence de 1 à 3 mm, et mieux de 2 mm. Le milieu dans lequel est réalisée l'observation est un milieu dispersant assurant une bonne dispersion des fibres rigides, par exemple de l'eau, un gel aqueux d'argile ou de polyuréthane associatif. On peut même réaliser une observation directe de la composition contenant les fibres rigides. Un échantillon de la composition ou de la dispersion préparée est placée entre lame et lamelle pour l'observation au microscope avec un objectif permettant un grossissement de 2,5 et avec une vision plein champ. La vision plein champ permet de voir les fibres dans leur intégralité.

Les fibres rigides peuvent être choisies parmi les fibres d'un polymère synthétique choisi parmi les polyesters, les polyuréthanes, les polymères acryliques, les polyoléfines, les polyamides, en particulier les polyamides non aromatiques et les polyimides-amides aromatiques.

Comme exemples de fibres rigides, on peut citer les fibres :
- de polyesters, telles que celles obtenues par découpe de fils vendus sous les dénominations FIBRE 255-100-R11-242T TAILLE 3 MM (section octalobée), FIBRE 265-34-R11-56T TAILLE 3 MM (section ronde), FIBRE COOLMAX 50-34-591 TAILLE 3 MM (section tétralobée) par la société DUPONT DE NEMOURS ;
- de polyamide, telles que celles vendues sous les dénominations TRILOBAL NYLON 0.120-1.8 DPF; TRILOBAL NYLON 0.120-18 DPF; NYLON 0.120-6 DPF par la société Cellusuede products ; ou obtenues par découpe de fils vendus sous la dénomination FIBRE NOMEX BRAND 430 TAILLE 3 MM par la société DUPONT DE NEMOURS ;
- de polyimide-amide telles que celles vendues sous les dénomination "KERMEL", " KERMEL TECH" par la société RHODIA ;
- de poly-(p-phénylène-téréphtalamide) (ou d'aramide) notamment vendues sous la dénomination Kevlar® par la société DUPONT DE NEMOURS ;
- des fibres à structure multicouche comprenant des couches alternées de polymères choisis parmi les polyesters, les polymères acryliques, les polyamides, telles que celles décrites dans les documents EP-A-6921217, EP-A-686858 et US-A-5472798. De telles fibres sont vendues sous les dénominations "Morphotex", « Teijin Tetron Morphotex » par la société TEIJIN.

Les fibres rigides particulièrement préférées sont les fibres de polymide-amide aromatiques.

Des fils ou fibres de polyimide-amide, qui peuvent être utilisés pour les compositions de l'invention, sont décrits, par exemple, dans le document de R. PIGEON et P. ALLARD, Chimie Macromoléculaire Appliquée, 40/41 (1974), pages 139-158 (n° 600), ou bien encore dans les documents US-A-3 802 841, FR-A-2 079 785, EP-A1-0 360 728, EP-A-0 549 494, auquels on pourra se référer.

Les fibres de polyimide-amide aromatique préférées sont des fibres de polyimide-amide comprenant des motifs répétitif de formule : 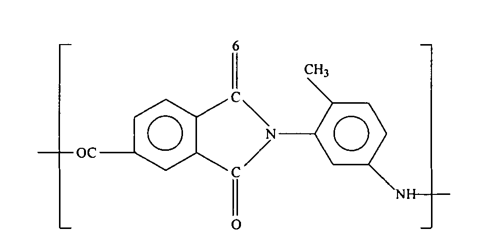 obtenu par polycondensation du toluylène diisocyanate et de l'anhydride triméllitique.

Les fibres peuvent êtres présentes dans la composition selon l'invention en une teneur allant de 0,1 % à 10 % en poids, par rapport au poids total de la composition, de préférence de 0,5% à 5% en poids, et mieux de 1 % à 3% en poids.

Les fibres utilisées dans la composition selon l'invention sont recouvertes d'au moins un azurant optique.

Les azurants optiques sont des composés bien connus de l'homme du métier. De tels composés sont décrits dans "Fluorescent Whitening Agent, Encyclopedia of Chemical Technology, Kirk-Othmer", vol 11, p. 227-241, 4^{ème} édition, 1994, Wiley.

On peut plus particulièrement les définir comme des composés qui absorbent essentiellement dans l'UVA entre 300 et 390 nm et réémettent essentiellement entre 400 et 525 nm.

Parmi les azurants optiques, on peut plus particulièrement citer les dérivés du stilbène, en particulier les polystyrylstilbènes et les triazinylstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et leurs mélanges.

De tels composés sont facilement disponibles commercialement. On peut citer par exemple :
- le dérivé stilbénique de naphto-triazole vendu sous la dénomination commerciale « Tinopal GS », le di-styryl-4,4' biphényle disulfonate di-sodique (nom CTFA : disodium distyrylbiphenyl disulfonate) vendu sous la dénomination commerciale « Tinopal CBS-X », le dérivé cationique d'aminocoumarine vendu sous la dénomination commerciale « Tinopal SWN CONC. », le 4,4'-bis[(4,6-dianilino-1,3,5-triazin-2-yl)amino]stilbène-2,2'-disulfonate de sodium vendu sous la dénomination commerciale « Tinopal SOP », le 4,4'-bis-[(4-anilino-6-bis(2-hydroxyéthyl)amino-1,3,5-triazin-2-yl)amino]stilbène-2,2'-disulfonic acide vendu sous la dénomination commerciale « Tinopal UNPA-GX », le 4,4'-bis-[4-anilino-6-morpholine-1,3,5-triazin-2-yl)amino]stilbène vendu sous la dénomination commerciale « Tinopal AMS-GX », le 4,4'-bis-[(4-anilino-6-(2-hydroxyéthyl)méthyl amino-1,3,5-triazin-2-yl)amino]stilbène-2,2'-disodium sulfonate vendu sous la dénomination commerciale « Tinopal 5BM-GX », tous par la société CIBA Spécialités Chimiques,
- le 2,5 thiophène di-yl bis(5 ter-butyl-1,3 benzoxazole) vendu sous la dénomination commerciale « Uvitex OB » par la société CIBA,
- le dérivé anionique du di-aminostilbène en dispersion dans l'eau vendu sous la dénomination commerciale « Leucophor BSB liquide » par la société CLARIANT,
- les laques d'azurant optique vendues sous la dénomination commerciale « COVAZUR » par la société WACKHERR.

Les azurants optiques utilisables dans la présente invention peuvent aussi se présenter sous la forme de copolymères, par exemple d'acrylates et/ou de méthacrylates, greffés par des groupement azurants optiques comme décrits dans la demande FR99-10942.

Les azurants optiques préférentiellement utilisés selon l'invention sont le di-styryl-4,4' biphényle disulfonate di-sodique, le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole) et leurs mélanges.

La composition selon l'invention peut comprendre un milieu solvant organique comprenant un solvant organique un mélange de solvants organiques, ou un milieu aqueux.

On peut citer comme solvant organique utilisable dans la composition :
- les cétones liquides à température ambiante, telles que la méthyléthylcétone, la méthylisobutylcétone, la diisobutylcétone, l'isophorone, la cyclohexanone, l'acétone ;
- les alcools liquides à température ambiante, tels que l'éthanol, l'isopropanol, le diacétone alcool, le 2-butoxyéthanol, le cyclohexanol ;
- les glycols liquides à température ambiante, tels que l'éthylène glycol, le propylène glycol, le pentylène glycol, le glycérol ;
- les éthers de propylène glycol liquides à température ambiante, tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total), tels que l'acétate d'éthyle, l'acétate de méthyle, l'acétate de propyle, l'acétate de n-butyle, l'acétate d'isopentyle ;
- les alcanes liquides à température ambiante, tels que le décane, l'heptane, le dodécane, le cyclohexane ;
- les aldéhydes liquides à température ambiante, tels que le benzaldéhyde, l'acétaldéhyde ; et
- leurs mélanges.

De préférence, le solvant est choisi parmi les esters à chaîne courte (ayant de 3 à 8 atomes de carbone au total), les alcools liquides à température ambiante, et leurs mélanges.

La composition à milieu solvant organique peut comprendre, en outre, de l'eau, notamment à une teneur allant de 0,1 à 10 % en poids, par rapport au poids total de la composition.

Lorsque la composition de vernis à ongles comprend un milieu solvant organique, le solvant organique peut être présent en une teneur allant de 5 à 95% en poids, par rapport au poids total de la composition, et de préférence de 50% à 70% en poids.

Lorsque la composition de vernis à ongles, comprend un milieu aqueux, ce dernier peut être constitué essentiellement d'eau (notamment le milieu aqueux est de l'eau) ou bien encore peut comprendre un mélange d'eau et d'un ou plusieurs solvants miscible(s) à l'eau, comme les monoalcools inférieurs ayant de 1 à 5 atomes de carbone, tels que l'éthanol, les glycols ayant de 2 à 8 atomes de carbone, les cétones en C₃-C₄ et les aldéhydes en C₂-C₄.
La teneur en eau de la composition peut aller de 5 à 95 % en poids par rapport au poids total de la composition, de préférence de 50 à 70% en poids.

La composition peut en outre comprendre au moins un polymère filmogène en une proportion allant généralement de 1 à 70 % en poids par rapport au poids total de la composition et mieux de 10 à 45% en poids dans un milieu solvant organique ou un milieu aqueux, tel que défini ci-dessus.

Dans la présente demande, on entend par « polymère filmogène » un polymère apte à former à lui seul ou en présence d'un agent plastifiant éventuel, un film isolable. Le polymère filmogène peut être solubilisé ou dispersé sous forme de particules dans le milieu cosmétiquement acceptable de la composition.

Le polymère filmogène peut être choisi parmi les polymères radicalaires, les polycondensats et les polymères d'origine naturelle.

Le polymère filmogène peut être choisi notamment dans le groupe formé par les polymères vinyliques et les acryliques, les polyuréthanes, les polyesters, les résines alkydes, les résines époxyesters, les polymères cellulosiques, tels que la nitrocellulose, les esters de cellulose comme l'acétate de cellulose, l'acétopropionate de cellulose, l'acétobutyrate de cellulose, les résines résultant de la condensation de formaldéhyde avec une arylsulfonamide et leurs mélanges.

Lorsque la composition comprend un milieu solvant organique, le polymère filmogène peut être choisi notamment parmi les résines alkydes, acryliques et/ou vinyliques, les polyuréthanes et les polyesters, les celluloses et dérivés cellulosiques, telles que la nitrocellulose, les esters de cellulose, tels que l'acétate de cellulose, l'acétopropionate de cellulose, l'acétobutyrate de cellulose, les résines résultant de la condensation de formaldéhyde avec une arylsulfonamide et leurs mélanges.

Lorsque la composition comprend un milieu aqueux, alors le polymère filmogène est généralement présent sous la forme de particules en dispersion dans le milieu aqueux formant ainsi un latex ou pseudo latex.

Parmi les polymères filmogènes susceptibles d'être utilisés en milieu aqueux, on peut citer les polyuréthanes, par exemple anioniques, les polyesters-polyuréthanes, les polyéther-polyuréthanes, les polymères radicalaires, notamment de type acrylique, acrylique styrène et/ou vinylique, les polyesters, les résines alkydes, seuls ou en mélange.
La dispersion peut également comprendre un polymère associatif de type polyuréthane ou une gomme naturelle, telle que la gomme xanthane.

Comme polymère en dispersion aqueuse, on peut citer les dispersions de polymères acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la Société ZENECA, DOW LATEX w432®, par la Société DOW CHEMICAL. On peut également employer des dispersions aqueuses de polyuréthane, et notamment les polyester-polyuréthanes vendus sous les dénominations « AVALURE UR-405® », « AVALURE UR-410® », « AVALURE UR-425® », « SANCURE 2060® » par la Société GOODRICH et les polyéther-polyuréthanes vendus sous les dénominations « SANCURE 878® » par la Société GOODRICH, « NEOREZ R-970® » par la Société AVECIA.

Le polymère filmogène peut être présent dans la composition selon l'invention en une teneur en matières sèches allant de 1% à 70% en poids, par rapport au poids total de la composition, et mieux de 10% à 45%.

Pour améliorer les propriétés filmogènes de la composition de vernis à ongle un agent auxiliaire de filmification peut être prévu.
Un tel agent auxiliaire de filmification peut être choisi parmi tous les composés connus de l'homme du métier, comme étant susceptibles de remplir la fonction recherchée, et être notamment choisi parmi les agents plastifiants.

En outre, lorsque la composition selon l'invention comprend un polymère filmogène sous forme de particules dispersées dans un milieu aqueux, l'agent auxiliaire de filmification peut aussi être choisi parmi les agents de coalescence.

La composition peut comprendre, en outre, au moins un agent plastifiant. En particulier, on peut citer, seuls ou en mélange, les plastifiants usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycol ;
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, des tartrates, des phosphates, des sébaçates ;
- des dérivés oxyéthylénés, tels que les huiles oxyéthylénées, notamment les huiles végétales, telles que l'huile de ricin ;
- leurs mélanges.

La quantité de plastifiant peut être choisie par l'homme du métier sur base de ses connaissances générales, de manière à obtenir une composition ayant des propriétés cosmétiquement acceptables. La teneur en plastifiant peut, par exemple, aller de 0,1 % à 15 % en poids, par rapport au poids total de la composition, et de préférence de 0,5 % à 10 % en poids.

La composition peut comprendre une matière colorante qui peut être choisie parmi les composés pulvérulents et/ou les colorants solubles dans le milieu de la composition. La matière colorante peut être présente en une teneur allant de 0,01 % à 10 % en poids, par rapport au poids total de la composition.

Les composés pulvérulents peuvent être choisis parmi les pigments et/ou les nacres et/ou les paillettes, habituellement utilisés dans les vernis à ongles.
Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique, les pigments métalliques comme l'aluminium, le cuivre ou le bronze. Parmi les pigments organiques, on peut citer le noir de carbone, les pigments de type D & C, et les laques à base de carmin de cochenille, de baryum, strontium, calcium, aluminium, la guanine.

On peut également citer les pigments à effet tels les particules comportant un substrat organique ou minéral, naturel ou synthétique, par exemple le verre, les résines acrylique, polyester, polyuréthane, polyéthylène téréphtalate, les céramiques, les alumines et recouvert ou non de substances métalliques comme l'aluminium, l'or , le cuivre, le bronze, ou d'oxydes métalliques comme le dioxyde de titane, l'oxyde de fer, l'oxyde de chrome, de pigments minéraux ou organiques et leurs mélanges.

Les pigments nacrés peuvent être choisis parmi les pigments nacrés blancs, tels que le mica recouvert de titane, ou d'oxychlorure de bismuth, les pigments nacrés colorés, tels que le mica titane avec des oxydes de fer, le mica titane avec notamment du bleu ferrique ou de l'oxyde de chrome, le mica titane avec un pigment organique du type précité ainsi que les fluorophlogopites avec des oxydes de fer.

On peut aussi utiliser des pigments à propriétés goniochromatiques, notamment à cristaux liquides ou multicouches.

Les colorants sont, par exemple, le rouge Soudan, le DC Red 17, le DC Green 6, le β-carotène, l'huile de soja, le brun Soudan, le DC Yellow 11, le DC Violet 2, le DC Orange 5, le jaune quinoléine.
La matière colorante peut également être choisie parmi les azurants optiques cités précédemment.

La composition peut comprendre, outre les fibres enrobées d'azurant optiques, des fibres non enrobées d'azurant optiques.

La composition peut comprendre, en outre, tout autre ingrédient, additif, utilisé couramment dans les compositions cosmétiques et connu de l'homme du métier comme étant susceptible d'être incorporé dans une composition de vernis à ongles.

De tels ingrédients, additifs, peuvent être choisis parmi les agents de coalescence, les agents épaississants, les conservateurs, les parfums, les huiles, les cires, les tensioactifs, les antioxydants, les agents antiradicaux libres, les agents d'étalement, les agents mouillants, les agents dispersants, les anti-mousse, les neutralisants, les stabilisants, les actifs choisis parmi les huiles essentielles, les filtres UV/solaires, les agents hydratants, les vitamines, les protéines, les céramides , les extraits végétaux , etc.. ; et leurs mélanges.

Bien entendu, l'homme du métier veillera à choisir ce ou ces éventuels composés complémentaires et/ou leur quantité, de manière telle que les propriétés de la composition selon l'invention ne soient pas, ou substantiellement pas altérées par l'adjonction envisagée.

L'exemple qui suit illustre de manière non limitative l'invention.

### Exemple 1 : Vernis à ongles

On a préparé un vernis à ongles ayant la composition suivante :

| | |
|---|---|
| Nitrocellulose | 10 g |
| Tributyl citrate | 5 g |
| Résine alkyde | 10 g |
| Pigments | 0,2 g |
| Agent épaississant | 1,5 g |
| Fibres recouvertes d'azurant optique (Fiberlon 54 ZO3 de la société LCW) | 5 g |
| Alcool Isopropylique | 5 g |
| Acétate d'éthyle | 20 g |
| Acétate de butyle | qsp 100 g |

### Mode opératoire :

La nitrocellulose est solubilisée dans le mélange solvant (acétate d'éthyle et acétate de butyle), puis le plastifiant (tributyl citrate) et la résine alkyde sont ajoutés à l'ensemble.
L'agents épaississant et les pigments sont dispersés et broyés à la broyeuse tri cylindre soit dans la totalité, soit dans une partie du mélange ci-dessus.
Les fibres sont ajoutées et dispersées dans le mélange sous forte agitation.

Après application sur les ongles, le film de vernis a été jugé comme présentant de bonnes propriétés de tenue et de résistance aux chocs et aux frottements, et ayant un effet réparateur de l'ongle.

### Exemple 2 : Composition de Base Traitante

On a préparé une base transparente traitante de vernis à ongles ayant la composition suivante :

| | |
|---|---|
| Nitrocellulose | 10 g |
| Acétyl tributyl citrate | 5 g |
| Résine alkyde | 10 g |
| Silice pyrogénée | 1,5 g |
| Fibres recouvertes d'azurant optique (Fiberlon 54 ZO3 de la société LCW) | 2 g |
| Alcool Isopropylique | 5 g |
| Acétate d'éthyle | 20 g |
| Acétate de butyle | qsp 100 g |

### Mode opératoire

La nitrocellulose est solubilisée dans le mélange solvant (acétate d'éthyle et acétate de butyle), puis le plastifiant (Acétyl tributyl citrate) et la résine alkyde sont ajoutés à l'ensemble.
L'agent épaississant et les pigments sont dispersés et broyés à la broyeuse tri cylindre soit dans la totalité, soit dans une partie du mélange ci-dessus.
Les fibres sont ajoutées et dispersées dans le mélange sous forte agitation.

Après application de la base traitante sur les ongles, le film a été jugé comme présentant de bonnes propriétés de tenue et comme conférant aux ongles un effet renforçateur, réparateur et peut être considéré comme un "pansement" de l'ongle.
Après application sur des ongles abimés, cette base traitante a été jugée comme conférant à ces ongles un aspect sain.

## Revendications

1. Composition de vernis à ongles comprenant, dans un milieu cosmétiquement acceptable, des fibres recouvertes d'au moins un azurant optique.

2. Composition selon la revendication précédente **caractérisée en ce que** les fibres sont choisies parmi les fibres de soie, de coton, de laine, de lin, des fibres de cellulose, de polyamide, de viscose, d'acétate notamment d'acétate de rayonne, de poly-p-(phénylène-téréphtalamide), en acrylique notamment de polyméthacrylate de méthyle ou de poly 2-hydroxyéthyl méthacrylate, de polyoléfine et notamment de polyéthylène ou de polypropylène, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères, les fibres rigides sensiblement rectilignes et leurs mélanges.

3. Composition selon l'une des revendications précédentes **caractérisée en ce que** les fibres sont des fibres d'origine synthétique.

4. Composition selon l'une des revendications précédentes **caractérisée en ce que** les fibres ont une longueur allant de 1 nm à 10 mm, de préférence de 1 µm à 10 mm, mieux de 0,1 mm à 5 mm et mieux de 0,3 à 1 mm.

5. Composition selon l'une des revendications précédente, **caractérisée en ce que** les fibres ont un section comprise dans un cercle de diamètre allant de 2 nm à 500 µm, de préférence allant de 100 nm à 100 µm.

6. Composition selon l'une des revendications précédentes, **caractérisée en ce que** les fibres sont présentes en une teneur allant de 0,1 % à 10 % en poids par rapport au poids total de la composition, de préférence de 0,5 à 5% en poids.

7. Composition selon l'une des revendications précédentes, **caractérisée en ce que** l'azurant optique est choisi parmi les dérivés du stilbène, les dérivés coumariniques, les dérivés oxazole, benzoxazole, imidazole, triazole, pyrazoline, les dérivés du pyrène et les dérivés de porphyrine et leurs mélanges.

8. Composition selon l'une des revendications précédentes, **caractérisée en ce que** l'azurant optique est choisi parmi le di-styryl-4,4' biphényle disulfonate di-sodique, le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole) et leurs mélanges.

9. Composition selon l'une des revendications précédentes, **caractérisée en ce qu'**elle comprend un polymère filmogène.

10. Composition selon l'une des revendications précédentes, **caractérisée en ce que** le polymère filmogène représente de 1 à 70% en poids par rapport au poids total de la composition et mieux de 10 à 45% en poids.

11. Composition selon l'une des revendications précédentes, **caractérisée en ce qu'**elle comprend un milieu solvant organique comprenant au moins un solvant organique.

12. Composition selon la revendication précédente, **caractérisée en ce que** le solvant organique représente de 5 à 95% en poids, par rapport au poids total de la composition, et de préférence de 50% à 70% en poids.

13. Composition selon l'une des revendications 1 à 10, **caractérisée en ce qu'**elle comprend un milieu aqueux.

14. Composition selon la revendication précédente, **caractérisée en ce que** le milieu aqueux représente de 5 à 95 % en poids par rapport au poids total de la composition, de préférence de 50 à 70% en poids.

15. Composition selon l'une des revendications précédentes, **caractérisée en ce qu'**elle comprend une matière colorante.

16. Composition selon la revendication précédente, **caractérisée en ce que** la matière colorante est présente en une teneur allant de 0,01 % à 10% en poids, par rapport au poids total de la composition.

17. Procédé cosmétique de maquillage ou soin non thérapeutique des ongles comprenant l'application sur les ongle d'au moins une couche d'une composition de vernis à ongles selon l'une des revendications 1 à 16.

18. Utilisation de fibres recouvertes d'au moins un azurant optique dans une composition de vernis à ongles, pour conférer au dépôt obtenu après l'application de la dite composition sur les ongles un aspect homogène et/ou régulier et/ou un camouflage des imperfections des ongles et/ou une bonne tenue et/ou une résistance mécanique du film de composition et/ou un effet renforçateur de l'ongle.
